Europäisches Patentamt

European Patent Office

Office européen des brevets

(11) Publication number: **0 222 419**
**A2**

## EUROPEAN PATENT APPLICATION

(21) Application number: 86200311.8

(22) Date of filing: 28.02.86

(51) Int. Cl.4: **G01N 33/49** , //G01N33/72

(30) Priority: 14.11.85 ES 548890

(43) Date of publication of application:
20.05.87 Bulletin 87/21

(84) Designated Contracting States:
AT BE CH DE FR GB IT LI LU NL SE

(71) Applicant: INSTITUTO DE BIOINGENIERIA,
FUNDACION CARLOS GARCIA-MONZON
General Rodrigo Street 6
E-28003 Madrid(ES)

(72) Inventor: De La Quintana, Joaquin Bustelo
Avda. de la Marina 3-5
Cadiz(ES)

(74) Representative: Modrie, Guy et al
Bureau Gevers SA 7, rue de Livourne Bte 1
B-1050 Bruxelles(BE)

(54) Process for quantifying labile glycosylated hemoglobin and glycemia.

(57) Process for quantifying the labile glycosylated hemoglobin and the glycemia in vivo, in a non invasive way, characterised in that two operative steps are provided therein, the first of which consists in a spectrophotometrical process using a source of light, the latter being applied through the skin in a non invasive way, and defining a spectrum of absorptions between two predetermined lengths of wave, in which spectrum of absorptions is defined a peak or maximum value of absorption which is deplaceable above the axis of wave lengths as a function of the labile glycosylated hemoglobin level, carrying out a fragmentation of the area defined by the absorption spectrum, into two semi-areas, and making a quotient in % between the difference between said semi-areas and the total area, as well as a proportionality between the values of said quotient and the amount of labile glycosylated hemoglobin, while in a second operative step, one carries out the processing of the absolute values obtained in the prior step, in order to obtain the corresponding values of glycemia.

## "Process for quantifying labile glycosylated hemoglobin and glycemia".

This invention relates to a process for carrying out the quantification of the labile glycosylated hemoglobin in vivo as well as of the glycemia also, allowing a constant control of the latter in diabetics, and with the special particularity that said quantification is carried out in a non invasive way.

As it is known, the partial or total operative atrophy of pancreas entails a reduction or an annihilation of the insulin brought to the blood stream and indispensable for controlling glycemia, namely the glucose level in blood, this level having to be comprised between 80 and 110 mgr/dl.

As it is also known, an excessive glucose level in blood is the source of multiple and various pathological processes while it has been possible to verify that some of them, apparently irreversible, are satisfactorily resolved when a permanent control of glycemia is established by means of a insulin admixture regulated in a similar way as the pancreas itself of the patient would do under normal conditions.

From the preceding, one can deduce the importance of being able to realize a correct quantification of glycemia in order to carry out the appropriate, suitably proportioned insulin admixtures and to correc the problematic consequence of the existence of a high glucose level in blood, already mentioned hereinbefore.

Presently by means of clinical analyses, it is possible to determine the correct quantification of glycemia, but this is made with invasive methods, which renders the system not very efficient due to the fact that in practice it is impossible to provide such analyses with the periodicity which would be desirable.

More particularly, the generally used quantification method consists of establishing the "glycemic profiles" of the patient, for which it is necessary to provide five blood takings, suitably distributed along the day, these takings being normally made in the finger tips, which is very painful for the patient.

It follows that normally also only one weekly glycemic profile or the sum of two weekly profiles is carried out.

In each case, the method is excessively traumatic for the patient and moreover the quantification is much far from an optimum control which should be made daily. Moreover the ideal situation would be that said quantification be carried out in a permanent way, namely in the same manner as made by the human system itself, in order to bring insulin at precise moments and also in proportions which are concrete and adapted to the effective needs.

Thus, the process proposed by this invention allows to carry out a permanent analysis for quantification of hemoglobin so that the practice of this process, in conjunction with the use of a supply container of insulin duly regulated according to the data instantaneously obtained in the quantification process, determines the obtention of an "artificial pancreas" which operates very closely as a natural pancreas.

To this end and in a more concrete manner, in the proposed process two steps are provided, one of quantification of the labile or rapid glycosylated hemoglobin and another step wherein the absolute values obtained from the first step are processed in order to obtain the corresponding values of glycemia.

The first step of the process is based on the fact that, as it is known, glucose is bonded to hemoglobin in a proportion varying as a function of the glycemia, giving rise to what is called glycosylated hemoglobin. A portion of the glucose is bonded to hemoglobin in a stable form but the other portion is of labile form, that is to say that this type of labile glycosylated hemoglobin varies in the blood in parallel relation with increases and decreases of the glycemia level.

Thus, starting from this fact, the first step of the process consists in a spectrophotometric process using a source of light, this latter being applied through the skin in a non invasive way, establishing a working zone between two predetermined lengths of wave and defining spectrum of absorptions, which increases from the lower length of wave and which after having defined a "peak" of maximum absorption, decreases to a point corresponding to the maximum length of wave.

It has been found that as a function of the labile glycosylated hemoglobin level in blood, there is a displacement of said peak or maximum value of absorption, in parallel relation with the axis corresponding to the magnitude of the wave lengths, and it has been possible to establish a proportionality between said variations of the spectrum and the amount of rapid or labile,glycosylated hemoglobin within some determined lengths of wave.

More precisely, the spectrum must be established between a minimum length of wave comprised between 520 and 575 nm, and a maximum length of wave comprised between 575 and 620 nm.

The absorptions between said lengths of wave are arranged by areas and semi-areas, the total area corresponding to that such as defined in the spectrum by the extreme lengths of wave, while the semi-areas are defined by an intermediate separation line corresponding to a length of wave comprised between 560 and 590 nm, it being obvious that for a preselected determined intermediate length of wave, the two semi-areas defined by the same will vary in real value as a function of the position which is occupied in the spectrum by the "peak" of maximum absorption, a determined relation between said areas and semi-areas being thus variable as a function of the position of said peak, allowing to establish a proportionality with respect to the amount of labile glycosylated hemoglobin.

More specifically, this relation is materialized by the quotient between the difference between the two semi-areas and the total area, in percentages.

The second operative step of the process consists in the processing of the absolute values resulting from the prior step, in order to obtain the corresponding values of glycemia, which does not raise any problem due to the fact that the proportion between the glycemia level and the labile glycosylated hemoglobin level has been perfectly defined by the present technology.

For carrying out the first operative step of the process and as it is obvious for any expert in spectrophotometry, one uses a source of light, a reticle of diffraction, slot filters, photosensors, a selector of parameters and a signal shaper. The source of light emits a radiation within the visible range, through the reticle of diffraction, which then is filtered in the above-mentioned slot filters, the latter directing said radiation till the photosensors which conform it, this radiation being then decoded and calibrated successively in order to pass to a selector of parameters and a device of final signal level evaluation.

Concerning the carrying-out of the second step of the process, as it will be evident for any expert in this field, one will provide a decoder, a calibration device for the normal conditions, a counter and a display, through which the obtained results will be visible for the operator.

It follows from what has been hereinabove explained that by the practical application of the process for quantification of labile glycosylated hemoglobin and glycemia, which forms the subject of this invention, it will be possible to make the control of said parameters in any diabetic with the frequences which is considered as suitable, including in a permanent way, and that in a non-invasive way, that is to say without the traditional takings of blood and without causing any other type of physical or psychological drawback for the patient.

According to a preferred practical embodiment of the process, the means for the cited carrying-out of the latter are embodied in a device that the patient will bear on himself permanently, suitably attached to the provided analysis zone, establishing a periodical quantification at relatively short time intervals, this device being combined with a container for injection of insulin, such as hereinabove mentioned, so that said container is activated by the signals received by the quantification device, and administrating insulin to the patient with the precise doses and at the appropriate moments according to the requirements of his glucose level in blood.

Those skilled in the art will easily understand the importance of the invention and the advantages thereof, it being understood that many changes and modifications may be provided without departing from the scope of this invention.

## Claims

1. Process for quantifying the labile glycosylated hemoglobin and the glycemia in vivo, in a non invasive way, characterised in that two operative steps are provided therein, the first of which consists in a spectrophotometrical process using a source of light, the latter being applied through the skin in a non invasive way, and defining a spectrum of absorptions between two predetermined lengths of wave, in which spectrum of absorptions is defined a peak or maximum value of absorption which is deplaceable above the axis of wave lengths as a function of the labile glycosylated hemoglobin level, carrying out a fragmentation of the area defined by the absorption spectrum, into two semi-areas, and making a quotient in % between the difference between said semi-areas and the total area, as well as a proportionality between the values of said quotient and the amount of labile glycosylated hemoglobin, while in a second operative step, one carries out the processing of the absolute values obtained in the prior step, in order to obtain the corresponding values of glycemia.

2. Process for quantifying the labile glycosylated hemoglobin and the glycemia in vivo, in a non invasive way according to claim 1, characterised in that the spectrum of analysed absorptions is established between a minimum length of wave comprised between 520 and 575 nm and a maximum length of wave comprised between 575 and 620 nm, while the fragmentation of said spectrum into the two semi-areas is made in correspondence with a length of wave comprised between 560 and 590 nm, the relation between both semi-areas varying for a determined spectrum and for a determined length of wave of fragmentation, as a function of the specific position occupied by the maximum absorption peak of the spectrum, this peak being displaceable as a function of the labile glycosylated hemoglobin in the blood.

3. Process for quantifying the labile glycosylated hemoglobin and the glycemia in vivo, in a non invasive way according to either of preceding claims, characterised in that for carrying out the first operative step of this process, one uses a source of light, a reticle of diffraction, slot filters, photosensors, a selector of parameters and a signal shaper, while for carrying out the second step of the process, one uses a decoder, a calibration device of the normal conditions, a counter and a display, through which the obtained results corresponding to the glycemia are made visible.